# EUROPEAN PATENT SPECIFICATION

(11) **EP 1 515 737 B1**
(45) Date of publication and mention of the grant of the patent: **12.10.2005**
(21) Application number: 03816108.9
(22) Date of filing: 23.06.2003
(51) Int. Cl.: A61K 35/78

(54) **AN AYURVEDIC NUTRITIONAL PREPARATION**
EINE AYURVEDISCHE NAHRUNGSERGÄNZUNGSZUSAMMENSETZUNG
PREPARATION NUTRACEUTIQUE AYURVEDIQUE

(30) Priority: 24.06.2002 IN MU05522002; 19.07.2002 IN MU06552002
(43) Date of publication of application: 23.03.2005
(73) Proprietor: Joseph, Anthony Devasia, Pune 411 042, Maharashtra (IN)
(72) Inventor: Joseph, Anthony Devasia, Pune 411 042, Maharashtra (IN)
(74) Representative: RACKETTE Partnerschaft Patentanwälte
(86) International application number: PCT/IN2003/000331
(87) International publication number: WO 2004/089385

(56) References cited:
- PATENT ABSTRACTS OF JAPAN vol. 1999, no. 14, 22 December 1999 (1999-12-22) & JP 11 255636 A (LION CORP), 21 September 1999 (1999-09-21)
- PATENT ABSTRACTS OF JAPAN vol. 010, no. 094 (C-338), 11 April 1986 (1986-04-11) & JP 60 224629 A (KAO SEKKEN KK), 9 November 1985 (1985-11-09)

## Description

**FIELD OF INVENTION:** The invention relates to an ayurvedic nutricinal preparation, essentially from nuts of the anacardiaciae family, Allium Sativum Linn and Zingiber Officinale Rosc for use as a prophylatic cardiac tonic, for general revitalization of the human body and for increasing cardiac muscle tonicity.

**BACKGROUND OF THE INVENTION:** Several ayurvedic preparations are in use for the treatment of heart ailments and as a prophylatic preparation for cardiac care. Most commonly used and reported are extracts or preparations from the plant Terminalia arjuna. Patent Application No: 1184/MUM/2001 describes a process for an ayurvedic preparation from Terminalia arjuna bark, Acorus calmus stem, Magnifera indica bark, Moringe oelifera bark, Murraya koengil leaf, Pipeer longumfruit, Boerhaavia diffusa root. The presently known ayurvedic preparations are very selectively effective.

The present invention describes for the first time a formulation for a complete ayurvedic preparation, made essentially from nuts of plants belonging to the genus anacardiaciae, for revitalising the human body. The preparation enhances the tonicity of the heart muscles, increases the production of blood by the marrow, reduces occlusion of arteries, improves myocardial blood flow and prevents cardiac ailments.

Nuts from the anacardiaciae family contain toxins and irritants, and cause contact dermatitis, because of which rash and irritation occurs. Hence its nutricinal value has been explored only in a limited way for cancer, gastric infections and fungal infections but not in respect of cardiovascular treatment. The invention also describes for the first time the use of nuts of the anacardiaciae family from which the toxic irritants are removed, and is combined with the extract of Allium Sativum Linn and Zingiber Officinale Rosc for treatment of cardiac ailments.

**SUMMARY:** In its main aspect the invention consists of an ayurvedic nutricinal preparation for use as a prophylactic for cardiac ailments and for increasing cardiac muscle tonicity . The principal ingredient used is a nut oil extract prepared from clean and dried nuts from plants of the genus anacardiaciae, and an extract of Allium Sativum Linn and Zingiber Officinale Rosc in vegetable oil . To obtain a nut oil extract, the nuts are cleaned and dried and the toxins and irritants contained in the nuts , are removed or deactivated by repeated cooking and drying of the nuts in milk to obtain pure detoxified nuts. The oil in the nuts which is the essential ingredient for this invention, is extracted by boiling the detoxified nuts in a medium of clarified butter, preferably obtained from cow's milk, until all the moisture is removed. In another aspect of this invention, 5 proportions by weight of ghee is used for every 2 proportions by weight of nuts. The medium is maintained at a temperature of 50° to 80°, for an extended period of time until the medium is absolutely moisture free. An extract of Allium Sativum Linn and Zingiber Officinale Rosc is obtained by cooking the two in a moisture free vegetable oil.. The nut oil extract and the Allium Sativum Linn and Zingiber Officinale Rosc extract are filtered separately and mixed together.

In another aspect of this invention, the nuts used are either of the species Semecarpous Anacardium Linn or Anacardium Occidenatale Linn.

In a further aspect of this invention Zingiber Officinale Rosc and Allium Sativum Linn are used in a proportion of 1:2 by weight in vegetable oil of 3 proportions by weight, and mixed with filtered nut oil extract obtained from 2 proportions by weight of nuts.

In yet another aspect of this invention the vegetable oil used is purified oil from Ricinus Communis Linn.

In a final aspect of this invention , the ayurvedic nutricinal preparation as described above is made available in soft gelatin capsules.

**DESCRIPTION:** The following description of the formulation is the best known method of working this invention.

In its main embodiment the invention consists of a nutticinal preparation for use as a prophylactic for cardiac elements and for increasing cardiac muscle tonicity.

It comprises essentially of
i) a nut oil extract in clarified butter. The nut oil extract is obtained from clean, dried and processed nuts from the plants of the genus anacardiaciae.
ii) An extract of Allium Sativum Linn and Zingiber Officinale Rose, which is prepared in moisture free vegetable oil

The nuts of the anacardiaciae family are cleaned and dried. Subsequently they are cooked in milk and dried . The process is repeated several times to obtain pure detoxified nuts from which the irritants and toxins have been removed or deactivated. Nuts preferably used are from the plants of Semecarpous Anacardium Linn or Anacardium Occidenatale Linn. The detoxified nuts are boiled in a medium of clarified butter (ghee), until all the moisture is removed and the spluttering of the medium ebbs. Clarified butter made from cow's milk is preferred. For every 2 proportion by weight of nuts, 5 proportion by weight of ghee is used. The medium is maintained at a temperature of 50° to 80° for an extended period of time to ensure removal of any remainder moisture. By the aforesaid process the essential oil from the nut is extracted in to the medium. By a separate process, Zingiber Officinale Rosc and Allium Sativum Linn in a proportion of 1:2 by weight is cooked in purified moisture free vegetable oil, preferably oil from Ricinus Communis Linn , to obtain a Zingiber Officinale Rosc - Allium Sativum Linn extract. The Zingiber Officinale Rosc - Allium Sativum Linn extract and the nut oil extract are separately filtered to remove all solid particles and mixed together.

In the preferred embodiment of this invention, 1:2 proportion by weight of Zingiber Officinale Rose and Allium Sativum Linn are cooked in 3 proportions by weight of vegetable oil, . The filtered extract of Zingiber Officinale Rosc and Allium Sativum Linn so obtained is mixed with filtered nut oil extract obtained from 2 proportions by weight of nuts.

The viscous liquid is ready for use in doses proportional to age and body weight of the consumer. The viscous liquid may also be encapsulated in soft gelatin capsules for dispensing.

The following are examples of some in vivo clinical trials conducted, on the basis of oral administration of said ayurvedic nutricinal preparation:

| **Comparative Study of I.H.D (Ischemic Heart Disease) Patient Before and** **After** **Administration of CF1 Based on Symptoms and 2D Echo / Color Doppler** | | | | | | | |
|---|---|---|---|---|---|---|---|
| **No** | **diagnosis** | **Symptoms pretreatment** | **Report of pretreatment 2D Echo** | **Period of treatment With CF1** | **Symptoms Post treatment** | **Report of post treatment 2D Echo** | **Remarks** |
| 001 | I.H.Dⁱ. | -Breathlessness on mild effort -Severe chest pain on walking -Giddiness | -Severe global hypokinesia -Severe LVⁱⁱⁱdysfunction -Severe PAH ^{viii}-LVEF^{iv} 15-20% | 1-0 X 90 | -NIL -Can walk comfortably 2-3 kms. | -Global hypokinesia reduced -Moderate LVⁱⁱⁱdysfunction -Mild PAH^{viii}-LVEF^{ix} 34% | -Overall good improvement |
| 003 | I.H.D. | -Angina on mild-effort -breathlessness on effort -unable to climb stairs | -Distal IVS^{x}, apex and anterior wall hypokinetic -Trivial MR^{v},TR^{vi}and PR^{vii}-LVEF^{iv} 50% | 1-0 X 60 | -Angina reduced -Breathlessness reduced to marked extent | -No regional wall motion abnormality at rest -Normal atrio-ventricular and semilunar valves -LVEF^{iv}60% Normal LVⁱⁱⁱsystolic function | -Overall good improvement -Maintained good health |
| 006 | I.H.Dⁱ. | -general debility -Chest pain and breathlessness on effort | -Distal IVS^{x} & LVⁱⁱⁱ is akinetic -LVEF^{iv}- 30% -Evidence of organised clot at LVⁱⁱⁱApex -LVⁱⁱⁱdiastolic dysfunction | 2-0 X 105 | -Breathlessness reduced -chest pain occassional -improved appetite -general feeling of well being | -LVEF -35% -Distal IVS^{x} & LVⁱⁱⁱ hypokinetic -No evidence of clot in LA^{ix}or LVⁱⁱⁱ-Normal LVⁱⁱⁱ dlastolic function | -Overall good improvement -Maintained good health |
| 011 | I.H.D. | -Chest discomfort -Pain in left arm -Weakness | -Ischemic heart disease -Hypokinetic inferior and apical segments -LVEF^{iv} 45% | 2-0 X 120 | -Chest discomfort reduced pain in the left arm disappeared -feeling of well being | -Normal sized LV -Normal systolic & diastolic function -LVEF^{iv} 78% No mitral valve prolapse -No segmental wall motion anomaly | -overall good recovery |
| 012 | I.H.D. | -Breathlessness -Chest pain on effort -Heart bum frequently | -Ischemic heart disease -LVEF^{iv}39% | 2-0 X 90 | -No breathlessness -No chest pain -No heart burn working 8-10 hrs a day | -Normal function LV -LVEF^{iv} 55% | -Overall good recovery |

| | | | | | | | |
|---|---|---|---|---|---|---|---|
| i. I.H.D ― Ischemic Heart Disease ii. LV - Left Ventricle | | | | | | | |
| iii. LVEF - Left Ventricle Hypertrophy | | | | | | | |
| iv. M.R.- Mitral Regurgitation | | | | | | | |
| v. T.R.- Tricuspid Regurgitation | | | | | | | |
| vi . P.R.- Pulmonary Regurgitation | | | | | | | |
| vii. P.A.H.- Pulmonary Artery Hypertrophy | | | | | | | |
| viii. LA- Left Auricle | | | | | | | |
| ix. I.V.S.- Inter Ventricular Septum | | | | | | | |

## Claims

1. An ayurvedic nutricinal preparation for use as a prophylactic for cardiac ailments and for increasing cardiac muscle tonicity comprising essentially of
a. a nut oil extract , with minimal or no toxins and irritants, obtained from cleaned , dried and processed nuts from plants of the genus anacardiaceae;
b. an extract of Allium Sativum Linn and Zingiber Officinale Rosc Linn

2. A medicinal preparation as described in claim 1 where in the nuts are either of the species Semecarpous Anacardium Linn or Anacardium Occidenatale Linn.

3. A medicinal preparation as described in claim 2, where in the nut extract is prepared in clarified butter.

4. A medicinal preparation as described in claim 3, where the nut oil extract in clarified butter is obtained by using 5 proportion by weight of clarified butter for every 2 proportion by weight of nuts.

5. A medicinal preparation as described in claim 4, where in the extract of Allium Sativum Linn and Zingiber Officinale Rosc is prepared by cooking Allium Sativum Linn and Zingiber Officinale Rosc in moisture free vegetable oil

6. A medicinal preparation as described in claim 5, where in the Zingiber Officinale Rosc and Allium Sativum Linn are used in a proportion of 1:2 by weight in vegetable oil of 3 proportions by weight, and mixed with filtered nut oil extract obtained from 2 proportions by weight of nuts.

7. A medicinal preparation as described in claim 6 which is in soft gelatin capsule form.

8. A medicinal preparation as described in claim 2 , prepared by a process comprising of steps including:
a. Partial or complete removal of toxins and irritants from cleaned and dried nuts from plants of the genus, anacardiaceae , by repeated cooking and drying of the nuts in milk to obtain pure detoxified nuts;
b. Boiling the detoxified nuts in a medium of clarified butter preferably from cow's milk and maintaining the medium at a temperature of 50° to 80°, for an extended period of time until the medium is absolutely moisture free to obtain a nut oil extract from the nuts;
c. Cooking Allium Sativum Linn and Zingiber Officinale Rosc in moisture free vegetable oil to obtain an Allium Sativum Linn and Zingiber Officinale Rosc extract ;
d. Separately filtering the nut oil extract and Allium Sativum Linn- Zingiber Officinale Rosc extract and mixing the two.

9. A process as described in claim 8 , where in the vegetable oil used is purified oil from Ricinus Communis Linn .

10. A process as described in claim 9, where in the nut oil extract in clarified butter is obtained by using 5 proportion by weight of clarified butter for every 2 proportion by weight of nuts.

11. A process as described in claim 10, where in the Zingiber Officinale Rosc and Allium Sativum Linn are used in a proportion of 1:2 by weight in vegetable oil of 3 proportions by weight, and mixed with filtered nut oil extract obtained from 2 proportions by weight of nuts.

## Patentansprüche

1. Eine ayurvedische Nährstoff - Zubereitung zur Verwendung als vorbeugendes Mittel bei Herzbeschwerden und zur Erhöhung der Tonizität des Herzmuskels, welche im Wesentlichen
a. einen Nussöl - Extrakt mit minimalen oder keinen Toxinen und Reizstoffen, erhalten aus gereinigten, getrockneten und verarbeiteten Nüssen von Pflanzen der Gattung Anacardiacee;
b. einen Extrakt von Allium Sativum Linn und Zingiber Officinale Rose Linn
umfasst.

2. Medizinische Zubereitung wie in Anspruch 1 beschrieben, worin die Nüsse entweder von der Sorte Semecarpous Anacardium Linn oder Anacardium Occidenatale Linn sind.

3. Medizinische Zubereitung wie in Anspruch 2 beschrieben, worin der Nuss - Extrakt in geklärter Butter hergestellt wird.

4. Medizinische Zubereitung wie in Anspruch 3 beschrieben, worin der Nussöl - Extrakt in geklärter Butter durch Verwendung von 5 Gewichtsanteilen geklärter Butter für jeweils zwei Gewichtsanteile Nüsse erhalten wird.

5. Medizinische Zubereitung wie in Anspruch 4 beschrieben, worin der Extrakt von Allium Sativum Linn und Zingiber Officinale Rose durch Kochen von Allium Sativum Linn und Zingiber Officinale Rose in feuchtigkeitsfreiem pflanzlichem Öl hergestellt wird.

6. Medizinische Zubereitung wie in Anspruch 5 beschrieben, worin Zingiber Officinale Rose und Allium Sativum Linn in einem Verhältnis von 1 : 2 Gewichtsanteilen in 3 Gewichtsanteilen pflanzlichem Öl benutzt werden und mit gefiltertem Nussöl - Extrakt gemischt werden, der aus zwei Gewichtsanteilen Nüssen erhalten wird.

7. Medizinische Zubereitung wie in Anspruch 6 beschrieben, welche in der Form einer Weichgelatine - Kapsel vorliegt.

8. Medizinische Zubereitung wie in Anspruch 2 beschrieben, hergestellt durch ein Verfahren, das die Schritte umfasst, die enthalten:
a. Teilweise oder vollständige Entfernung von Toxinen und Reizstoffen aus gereinigten und getrockneten Nüssen von Pflanzen der Gattung Anacardiacee durch wiederholtes Kochen und Trocknen der Nüsse in Milch, um reine entgiftete Nüsse zu erhalten;
b. Kochen der entgifteten Nüsse in einem Medium von geklärter Butter, vorzugsweise aus Kuhmilch, und Halten des Mediums bei einer Temperatur von 50° bis 80° für einen ausgedehnten Zeitraum, bis das Medium vollständig frei von Feuchtigkeit ist, um aus den Nüssen einen Nussöl - Extrakt zu erhalten;
c. Kochen von Allium Sativum Linn und Zingiber Officinale Rose in feuchtigkeitsfreiem pflanzlichen Öl, um einen Extrakt von Allium Sativum Linn und Zingiber Officinale Rose zu erhalten;
d. getrenntes Filtrieren des Nussöl - Extraktes und des Extraktes von Allium Sativum Linn und Zingiber Officinale Rose und Mischen der beiden.

9. Verfahren wie in Anspruch 8 beschrieben, worin das pflanzliche Öl gereinigtes Öl aus Ricinus Communis Lin ist.

10. Verfahren wie in Anspruch 9 beschrieben, worin der Nussöl-Extrakt in geklärter Butter unter Verwendung von 5 Gewichtsanteilen geklärter Butter für jeweils 2 Gewichtsanteile Nüsse erhalten wird.

11. Verfahren wie in Anspruch 10 beschrieben, worin Zingiber Officinale Rose und Allium Sativum Linn in einem Verhältnis von 1 : 2 Gewichtsanteilen in 3 Gewichtsanteilen pflanzlichem Öl verwendet werden und mit dem filtrierten Nussöl - Extrakt, der aus 2 Gewichtsanteilen Nüsse erhalten wurde, gemischt werden.

## Revendications

1. Préparation nutritionnelle ayurvédique pour être utilisée comme substance prophylactique pour des maladies cardiaques et pour augmenter la tonicité du muscle cardiaque comprenant essentiellement
a) un extrait d'huile de noix, avec un minimum ou pas de toxines ni d'irritants, obtenu à partir de noix lavées, séchées et transformées en provenance de plantes du genre anacardiaceae ;
b) un extrait d'Allium Sativum Linn et de Zingiber Officinale Rosc Linn.

2. Préparation médicinale selon la revendication 1 dans laquelle les noix sont soit de l'espèce Semecarpous Anacardium Linn soit de l'espèce Anacardium Occidenatale Linn.

3. Préparation médicinale selon la revendication 2, dans laquelle l'extrait de noix est préparé dans du beurre clarifié.

4. Préparation médicinale selon la revendication 3, dans laquelle l'extrait d'huile de noix dans le beurre clarifié est obtenu en utilisant 5 parties en poids de beurre clarifié pour 2 parties en poids de noix.

5. Préparation médicinale selon la revendication 4, dans laquelle l'extrait d'Allium Sativum Linn et de Zingiber Officinale Rosc est préparé en cuisant l'Allium Sativum Linn et le Zingiber Officinale Rosc dans de l'huile végétale exempte d'humidité.

6. Préparation médicinale selon la revendication 5, dans laquelle le Zingiber Officinale Rosc et l'Allium Sativum Linn sont utilisés dans un rapport pondéral de 1/2 dans 3 parties en poids d'huile végétale, et mélangés avec l'extrait d'huile de noix filtré obtenu à partir de 2 parties en poids de noix.

7. Préparation médicinale selon la revendication 6, laquelle est sous forme de capsule molle.

8. Préparation médicinale selon la revendication 2, préparée suivant un procédé comprenant les étapes consistant à :
a) éliminer partiellement ou complètement les toxines et les irritants à partir de noix lavées et séchées en provenance de plantes du genre anacardiaceae, par cuisson et séchage répétés des noix dans du lait afin d'obtenir des noix pures et exemptes de toxines ;
b) faire bouillir les noix exemptes de toxines dans un milieu à base de beurre clarifié de préférence à base de lait de vache et maintenir le milieu à une température de 50 à 80°C, pendant une période de temps prolongée jusqu'à ce que le milieu soit absolument exempt d'humidité afin d'obtenir un extrait d'huile de noix à partir des noix ;
c) cuire l'Allium Sativum Linn et le Zingiber Officinale Rosc dans de l'huile végétale exempt d'humidité afin d'obtenir un extrait d'Allium Sativum Linn et de Zingiber Officinale Rosc ;
d) filtrer séparément l'extrait d'huile de noix et l'extrait de d'Allium Sativum Linn et de Zingiber Officinale Rosc et mélanger les deux.

9. Procédé selon la revendication 8, dans laquelle l'huile végétale utilisée est de l'huile purifiée de Ricinus Communis Linn.

10. Procédé selon la revendication 9, dans lequel l'extrait d'huile de noix dans le beurre clarifié est obtenu en utilisant 5 parties en poids de beurre clarifié pour 2 parties en poids de noix.

11. Procédé selon la revendication 10, dans lequel le Zingiber Officinale Rosc et l'Allium Sativum Linn sont utilisés dans un rapport pondéral de 1/2 dans 3 parties en poids d'huile végétale, et mélangés avec l'extrait d'huile de noix filtré obtenu à partir de 2 parties en poids de noix.
